(19) Europäisches Patentamt
European Patent Office
Office européen des brevets

(11) Numéro de publication: **0 179 751**
**B1**

(12) **FASCICULE DE BREVET EUROPÉEN**

(45) Date de publication du fascicule du brevet: **06.04.88**

(21) Numéro de dépôt: **85870125.3**

(22) Date de dépôt: **17.09.85**

(51) Int. Cl.⁴: **C 07 D 493/10**, C 12 P 17/18, A 61 K 31/35, A 61 K 31/70, A 23 K 1/16 // C12R1:47, C12P19:44, C12P17:18, C07D493:10, C07D311:00, C07D307:00

(54) **Ionophore polyéther, son procédé de préparation et son utilisation.**

(30) Priorité: **19.09.84 FR 8414375**

(43) Date de publication de la demande: **30.04.86 Bulletin 86/18**

(45) Mention de la délivrance du brevet: **06.04.88 Bulletin 88/14**

(84) Etats contractants désignés: **AT BE CH DE FR GB IT LI LU NL SE**

(56) Documents cité:
**EP-A-0 011 859**
**EP-A-0 070 669**
**DD-A-207 222**
**FR-A-2 097 053**

**CHEMICAL ABSTRACTS, vol. 101, 1984, page 537, résumé no. 169077v, Columbus, Ohio, US**

(73) Titulaire: **SANOFI, 40, Avenue George V, F-75008 Paris (FR)**
Titulaire: **CENTRE NATIONAL DE LA RECHERCHE SCIENTIFIQUE (CNRS), 15, Quai Anatole France, F-75007 Paris (FR)**

(72) Inventeur: **David, Lucien, Les Queilles- Orcet 7, F-63670 Le Cendre (FR)**
Inventeur: **Leal Ayala, Humberto, Estefania Castaneda 416 Col. Riberena, C. Reynosa, Tam. (MX)**
Inventeur: **Puygrenier, Marc, Avenue Jean Grandel 26, F-94360 Bry- Sur- Marnes (FR)**

(74) Mandataire: **Cauchie, Daniel, c/o S.A. LABAZ-SANOFI N.V. Avenue De Béjar, 1, B-1120 Bruxelles (BE)**

EP 0 179 751 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1988

**Description**

La présente invention se rapporte, d'une manière générale, à un nouvel ionophore polyéther ainsi qu'à son procédé de préparation.

En particulier, l'invention concerne l'ionophore polyéther sous forme acide appelé "abiérixine", répondant à la formule :

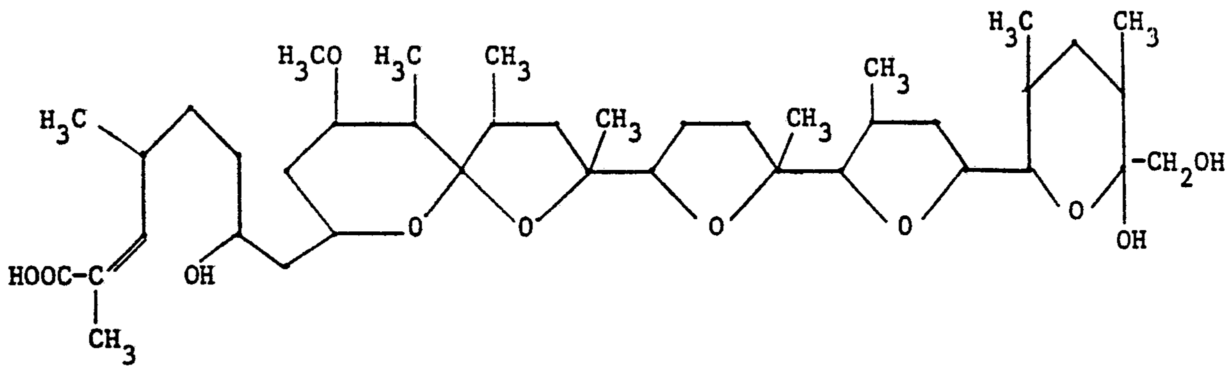

De même l'invention se rapporte à l'abiérixine sous forme hydratée ou sous forme de sels non toxiques.

Par sels non toxiques, on entend plus particulièrement les sels de métaux alcalins, tels que lithium, sodium ou potassium, les sels de métaux alcalino-terreux, tels que calcium ou magnésium, ou les sels d'ammonium, tels que ceux obtenus à partir de l'ammoniac ou d'une amine, telle que méthylamine, éthylamine, diméthylamine, diéthylamine, triéthylamine, éthanolamine, diéthanolamine, trométhamol.

On a trouvé que l'abiérixine et ses sels non toxiques sont doués de propriétés anticoccidiennes notamment vis-à-vis d'Eimeria tennella.

De même, les composés de l'invention se sont révélés utiles comme facteur de croissance chez les ruminants.

En outre, on a trouvé que les composés de l'invention sont capables d'exalter l'activité cytotoxique d'immunotoxines.

Un autre objet de l'invention concerne, par conséquent, une composition, en particulier une composition pharmaceutique ou une composition vétérinaire alimentaire ou thérapeutique contenant, comme principe actif essentiel, au moins un composé de l'invention en association avec un véhicule, diluant ou excipient approprié.

On connait déjà de nombreux antibiotiques du groupe des polyéthers par exemple la monensine, la nigéricine, la grisorixine, la salinomycine etc... ces composés étant actifs contre les bactéries Gram-positives, les champignons et les protozoaires. Ces antibiotiques montrent en outre des propriétés anticoccidiennes.

Par exemple, on a décrit dans les brevets de l'Allemagne de l'Est Nos. 208 172 et 237 725 des complexes d'antibiotiques produits à partir de souches de Streptomyces hygroscopicus. Ces composés polyéthers sont caractérisés par une activité antibiotique importante et comparativement plus importante que celle présentée par la monensine ou la salinomycine. Par contre, l'abiérixine est douée d'activité antibiotique très faible par rapport à celle présentée par la plupart des ionophores polyéthers connus comme la monensine, la nigéricine ou la grisorixine. Par exemple, la concentration minimale inhibitrice de l'abiérixine vis-à-vis de Bacillus cereus déterminée selon la méthode de dilution est de 25 µg/ml mais de 0,05 µg/ml, 0,05 µg/ml et 3,12 µg/ml dans le cas respectivement de la monensine, de la nigéricine et de la grisorixine.

En tant que facteur de croissance chez les ruminants, l'abiérixine présentera par conséquent, un avantage incontestable par rapport aux antibiotiques polyéthers connus car ce composé sera moins susceptible de provoquer une destruction de la flore du rumen.

Selon l'invention, on prépare l'abiérixine par culture d'une souche d'Actinomycetales et ce, en milieu nutritif contenant une source assimilable de carbone, d'azote et de sels minéraux.

Généralement, la souche utilisée est de l'espèce Streptomyces en particulier de la classe des hygroscopicus. A titre d'exemple, on peut citer, comme souches productrices d'abiérixine, Streptomyces hygroscopicus NRRL B 1865 et Streptomyces hygroscopicus NRRL 3602.

Une souche particulièrement utile pour la production d'abiérixine est la souche de Streptomyces déposée auprès de l'INSTITUT PASTEUR (Paris) dans la Collection Nationale de Cultures de Microorganismes (C.N.C.M.) sous le No. I-321. (Date de dépôt 31 juillet 1954).

Cette souche présente les caractéristiques suivantes :

I. Caractéristiques culturelles

En utilisant les milieux définis ci-après :

A : milieu extrait de malt et de levure (ISP 2)
B : milieu farine d'avoine (ISP 3)
C : milieu de BENNET (S.A. WAKSMANN "The actinomycetes" vol. 2 p. 33)
D : milieu d'EMERSON id. p. 331
E : milieu CZAPEK - saccharose id. p. 328

F : milieu CZAPEK - glycérol id. p. 328

G : milieu tyrosine de GORDON et MMITH [Jr. Bact. 69, 147-150 (1955)] on a relevé les caractéristiques culturelles ci-après :

| Milieu | Mycélium primaire | Sporulation | Pigment soluble |
|---|---|---|---|
| A | ivoire clair | blanc-gris* | néant |
| B | ivoire clair | grise* | néant |
| C | ivoire clair | pauvre | néant |
| D | ivoire clair | pauvre | néant |
| E | incolore | blanche* | néant |
| F | incolore | blanche* | néant |
| G | jaune pâle | blanche* | néant |

* très bonne pousse.

Sur tous les milieux, le mycélium devient pratiquement noir et humide mais seulement au bout de cinq ou six semaines.

Les spores forment des chaînes spiralées en spirales compactes. Leur surface est lisse.

II. Caractéristiques physiologiques

Croissance sur :

| | |
|---|---|
| - glucose | bonne |
| - arabinose | bonne |
| - xylose | bonne |
| - inositol | bonne |
| - mannitol | bonne |
| - fructose | bonne |
| - rhamnose | bonne |
| - sucrose | bonne |
| - raffinose | bonne |
| Réduction des nitrates | positive |
| Liquéfaction de la gélatine | positive |
| Formation de mélanine | négative |
| Réaction à la tyrosine | négative |
| Coagulation et peptonisation du lait | douteuse |
| Hydrolyse de l'amidon | positive |
| Production d'$H_2S$ | négative |
| Inhibition par la streptomycine | positive |

Les sucres mentionnés ci-dessus sont utilisés dans la classification de NONOMURA, J. Ferment. Technol. 52, 78-92 (1974).

D'autres caractéristiques des souches productrices d'abiérixine peuvent être relevées à partir du Tableau suivant :

**Tableau**

| Souche | Spores/ml (1) | Coleur du mycélium (2) | Sensibilité à la nigéricine (C.M.I) (3) | Production de : Nigéricine | Abiérixine |
|---|---|---|---|---|---|
| Streptomyces C.N.C.M.-I-321 | $2.10^7$ | blanc/gris | > 500 | + + | + |
| Streptomyces hygroscopicus NRRL B 1865 | $5.10^7$ | blanc/gris | > 500 | + + | + |
| Streptomyces hygroscopicus NRRL 3602 | $10^7$ | blanc/gris | > 500 | + + | + |

(1) : milieu gelosé contenant :
20g de glucose
10g de farine de soya
3g de $CaCO_3$
15g d'agar
eau q.a. 1000 ml
pH ajusté à 7

(2) : observations sur le milieu (1) ci-dessus jusqu'à 9 jours d'incubation
(3) : C.M.I. : concentration minimale inhibitrice exprimée en µg/ml.

Par contre le Streptomyces albua NRRL B 1685 ne produit ni nigéricine ni abiérixine dans les mêmes conditions que celles utilisés précédemment.

Ces réaultats laissent supposer qu'une souche productrice de nigéricine serait également productrice d'abiérixine.

Mode de réalisation de l'invention

Suivant un mode de réalisation pour l'obtention de l'abiérixine, on prépare d'abord un inoculum végétatif de Streptomyces par exemple Streptomyces C.N.C.M. - I-321 en ensemençant une petite quantité du bouillon de culture avec une suspension de spores congelés du micro-organisme.

On cultive ensuite ce micro-organisme sous conditions d'aérobiose, à une température comprise entre 25°C et 30°C, de préference à 27°C et sur différents milieux nutritifs contenant des éléments habituellement utilisés à cet effet.

En général, la production maximale d'abiérixine a lieu en 3 à 10 jours après l'inoculation du milieu de culture sous conditions d'aérobiose et d'agitation suffisantes.

Comme source de carbone, on peut utiliser le glucose, les dextrines, l'amidon, les mélasses.

Les sources d'azote peuvent être des extraits de levure, des extraits de viande, des hydrolysats de farine de soya, des hydrolysats de caséine, des peptones de viande, du "corn-steep" etc...

Avant l'inoculation par le micro-organisme, il est souhaitable d'ajuster le pH du milieu de culture entre environ 6,5 et 7,5 de préférence à 7,2 et, ce, au moyen d'un hydroxyde de métal alcalin, tel que l'hydroxyde de sodium ou de potassium. La fraction abiérixine produite lors de la fermentation est contenue dans le mycélium. Le meilleur rendement est obtenu à partir d'un mycélium isolé à l'aide d'une centrifugeuse et essoré.

On extrait alors l'abiérixine produite, par exemple au moyen d'éthanol, pendant 2 heures environ. On a remarqué que cette durée permet d'obtenir 98 % du poids de composés actifs produits lors de la fermentation constitués notamment d'abiérixine et de nigéricine. On purifie le mélange de polyéthers polycycliques ainsi obtenu, au moyen de trois chromatographies successives, d'abord par chromatographie sur colonne, de manière à éliminer les acides gras et la majeure partie de la nigéricine, ensuite par chromatographie-éclair (J. Org. Chem. 1978, 14, 2923) de manière à éliminer la totalité de l'élaiophyline et une partie de la nigéricine et finalement par chromatographie liquide haute pression (CLHP).

A ce stade, on isole l'abiérixine sous forme de sel de métal alcalin.

L'abiérixine elle-même peut être obtenue sous forme monohydratée par acidification de son sel de métal alcalin présent dans les fractions enrichies obtenues après purification par chromatographie sur colonne comme décrit précédemment.

Ces fractions enrichies, lorsqu'elles sont éluées en chromatographie-éclair au moyen de solvants contenant un acide, par exemple l'acide acétique fournissent l'abiérixine monohydratée.

Toutefois, on a remarqué que le traitement par l'acide chlorhydrique N/10 d'une solution éthérée du sel de sodium de l'abiérixine ne conduit pas à l'acidification totale de l'abiérixine.

Le monohydrate ainsi obtenu peut être chauffé modérément pour recouvrer l'abiérixine.

Quant aux sels non toxiques d'abiérixine, ceux-ci peuvent être produits de manière connue par réaction de l'abiérixine ou de son hydrate avec une base organique ou inorganique appropriée, telle qu'un hydroxyde de métal alcalin, un oxyde de métal alcalino-terreux, l'ammoniac ou une amine.

On a déterminé certaines propriétés physico-chimiques des composés de l'invention, à savoir :

A. Caractéristiques physico-chimiques de l'abiérixine monohydratée

Aspect : solide

P.F. : 83-85°C

Solubilité : se dissout dans les solvants organiques usuels, tels que les alcools inférieurs par exemple méthanol, éthanol, les esters inférieurs, par exemple l'acétate d'éthyle ou encore dans l'éther éthylique et le chloroforme.

Dans l'eau, la solubilité est de 44 % environ.

$\alpha_J^{25}$ : +45° (c = 0,03 n, méthanol)

Masse moléculaire : 742 déterminée par la méthode FAB (Fast Atom Bombardment) [Org. Mass Spectrum, 16, 256 (1981)].

L'ion (M-H)m/z : 723 apparaît dans le spectre FAB négatif et confirme la masse moléculaire m/z = 724 de l'abiérixine.

Formule brute : $C_{40}H_{68}O_{11} \cdot H_2O$

Analyse élémentaire :

| | C | H | O |
|---|---|---|---|
| Calculé pour $C_{40}H_{70}O_{12}$ : | 64,66 % | 9,49 % | 25,84% |
| Trouvé : | 64,41 % | 9,46 % | 25,60 % |

Spectre I.R. (pastille de KBr)

En infrarouge, le spectre de l'abiérixine, représenté à la figure 1, présente les bandes caractéristiques suivantes* :

3360(F), 2980(F), 2940(F), 2880(F), 2640(t.f.), 1730(f), 1640(f), 1460(m), 1370(m), 1360(e), 1355(e), 1310(e), 1300(e), 1290(f), 1260(t.f.), 1240(t.f.), 1205(t.f), 1185(t.f.), 1115(m), 1080(F), 1070(F), 1040(F), 1030(F), 1020(F), 990(f), 980(f), 960(m), 930(t.f.), 910(t.f.), 880(t.f.), 870(t.f.), 845(t.f.), 810(t.f.), 730(t.f.), 715(t.f.), 690(t.f.), 640(t.f.), 620(t.f.), 610(t.f.), 590(t.f.), 535(t.f.), 500(t.f.), 460(t.f.), 455(t.f.), 415(t.f.), 395(t.f.)$cm^{-1}$.

* : e = épaulement, t.f. = très faible, f = faible, m = moyen, F = forte.

De ces données, on peut tirer les informations suivantes :

- entre 3700 et 3150 $cm^{-1}$, une bande intense qui se prolonge jusqu'a 2500 $cm^{-1}$ implique la présence d'un ou plusieurs groupements hydroxylés dont un pourrait être un OH d'acide.
- trois bandes très intenses avec des maxima à 2970, 2940 et 2880 $cm^{-1}$ correspondent aux vibrations d'élongation des liaisons CH de la molécule.
- une bande intense à 1715 - 1690 $cm^{-1}$ que l'on peut attribuer aux vibrations de valence d'une liaison C=O d'acide conjugué du type $C{=}C\text{-}CO_2H$
- une bande intense a 1460 $cm^{-1}$ attribuable aux vibrations des déformations des liaisons C-H
- enfin, une bande large représentant plusieurs maxima entre 1115 et 1020 $cm^{-1}$ qui se rapporte a des vibrations de valence C-O-C.

Spectre U.V. (cyclohexanne)

En ultraviolet, le spectre de l'abiérixine, représenté à la figure 2 présente une bande intense ($\lambda$ max = 217 nm, $\varepsilon$ = 10400), caractéristique d'un groupement acide $\alpha,\beta$-insaturé, et une bande faible ($\lambda$ max , 275 nm, $\varepsilon$ = 2800).

Spectre R.M.N. ($CDCl_3$)

La figure 3 représente à 200 MHz le spectre de résonance magnétique nucléaire du proton de l'abiérixine (substance de référence : tétraméthylsilane) et les figures 4 let 5 représentent à 50,3 MHz le spectre de résonance magnétique nucléaire du $^{13}C$ de ce composé (substance de référence : tétraméthylsilane).

B. Caractéristiques physico-chimiques du sel de sodium de l'abiérixine.

Aspect : mousse blanche solide.

P.F. : 120-123°C

Solubilité : se dissout dans les solvants organiques usuels, tels que les alcools inférieurs par exemple méthanol, éthanol, les esters inférieurs par exemple l'acétate d'éthyle ou encore dans l'éther éthylique et le chloroforme.

Par contre, il est insoluble dans l'eau.

Spectre I.R. (KBr)

Le spectre infrarouge du sel de sodium de l'abiérixine se distingue de celui de l'abiérixine même par le fait que la bande intense à 1715 - 1690 $cm^1$ due à la liaison C=O de l'acide est remplacée par une bande intense à 1540 $cm^{-1}$ due à l'ion carboxylate.

On a décrit ci-dessus la préparation, selon l'invention, de l'abiérixine à partir de souches de Streptomyces.

Il est entendu que l'invention ne se limite pas à la production d'abiérixine à partir de ces souches mais englobe également des modes de réalisation équivalents à partir notamment des souches en question en particulier à partir de la souche Streptomyces C.N.C.M.-I-321 sans pour autant sortir du cadre de l'invention.

De tels mutants peuvent être obtenus par des techniques connues, par exemple en soumettant le micro-organisme producteur d'abiérixine à une irradation aux rayons X ou ultraviolets ou à l'action d'agents chimiques.

La coccidiose est une maladie commune et répandue chez la volaille, le porc, le lapin, le chat, le chien, chez certains ruminants etc..., maladie causée par une ou plusieurs espèces parasites du type protozoaire de souche Eimeria tels que E. tenella, E. necatrix, E. acervulina, E. maxima, E. brunetti, E. mivati, E. adenoides et E. maleagrimitis.

E. tenella et E. acervulina sont les agents responsables d'une infection importante et souvent fatale des poulets, infection qui se manifeste par une hémorragie sévére et importante, une accumulation de sang dans le caecum et par passage de sang dans les matières fécales.

E. necatrix attaque l'intestin grêle du poulet causant une coccidiose intestinale tandis que E. maleagrimitis et E. adenoides sont responsables de la coccidiose chez le dindon.

Laissées sans traitement, les infections graves dues à la coccidiose conduisent à un gain de poids inférieur à la normale, à une efficacité réduite de la nourriture et à une mortalité élevée chez les oiseaux.

Par conséquent, la morbidité et la mortalité occasionnées par les infections de coccidiose créent une perte économique importante lorsque les infections sont laissées sans traitement ou sans surveillance.

L'élimination ou le contrôle de cette maladie est, par conséquent, de première importance, notamment pour l'élevage de la volaille.

On a trouvé, dans le cadre de la présente invention, que l'abiérixine ainsi que ses sels non toxiques présentent de très intéressantes propriétés anticoccidiennes susceptibles d'être utilisées pour la destruction de parasites chez les oiseaux et certains ruminants (ovins).

Des tests ont été effectués de manière à montrer l'efficacité de l'abiérixine comme agent anticoccidien dans lesquels on a relevé les résultats zootechniques (poids, indice de consommation) et les symptômes chimiques de coccidiose (morbidité, mortalité, aspect des matières fécales, excrétion ookystale, lésions).

On a utilisé à cet effet le protocole expérimental suivant :

Animaux

- Coquelets de souche génétique Warren Sex Sal Link
- Age le jour de l'inoculation (J O) : 17 jours
- Age le jour de l'abattage (J+8) : 25 jours

Inoculum

Dose : 300 000 oocystes sporulés d'Eimeria tenella

Alimentation

- Aliment utilisé : aliment type poulet de chair, 1er âge
- Anticoccidien utilisé : l'abiérixine à la dose de 40 ou 50 ppm/jour depuis J-2 jusqu'à J+8

Lots

- Nombre d'animaux par lot : 4
- Répartition des lots :

NCNT : animaux non contaminés, non traités

CTNT : animaux contaminés, non traités

$CTT_1$ : animaux contaminés, traités avec 40 ppm d'abiérixine

$CTT_2$ : animaux contaminés, traités avec 50 ppm d'abiérixine

Les critères

- Les gains de poids (GP) de J-2 à J+8
- L'excrétion ookystale (EO) journalière de J+4 à J+8
- La morbidité (M) notée de 0 à 4
- L'aspect des fécès noté de 0 : fécès normales à 4 : fécès diarrhéiques ou hémorragiques (MF)
- La mortalité (M %) exprimée en pourcentage
- Les lésions (L) notées selon la notation de RFID (W. Malcolm REID, "A diagnostic chart of nine species of fowl coccidia - Georgia Agricultural Experiment Stations - Dec. 1964, Technical Bulletin N. S. 39)
- Les indices de consommation (IC)

**Tableau**

| Traitements<br>Critères | NCNT | | CTNT | | $CTT_1$ | | $CTT_2$ | |
|---|---|---|---|---|---|---|---|---|
| Poids moyen de départ | P | GP | P | GP | P | GP | P | GP |
| à J - 2 : 127 g | 286 g | 159 g | 199 g | 72 g | 219 g | 93 g | 190 g | 63 g |
| EO x $10^3$ | | | | | | | | |
| Total | 0 | | 5628 | | 4165 | | 2324 | |
| % | - | | - | | 74,0 | | 41,3 | |
| M | | | | | | | | |
| J + 5 | 0 | | 4 | | 3 | | 3 | |
| J + 6 | 0 | | 2 | | 0 | | 0 | |
| J + 7 | 0 | | 1 | | 0 | | 0 | |
| MF | | | | | | | | |
| J + 5 | 0 | | 4 | | 4 | | 3 | |
| J + 6 | 0 | | 2 | | 1 | | 1 | |
| J + 7 | 0 | | 2 | | 0 | | 0 | |
| M % | 0 | | 0 | | 0 | | 0 | |
| Lésion individuelle<br>Moyenne | 0 | | 3,5 | | 2,5 | | 3,5 | |
| IC de J - 2 à J + 8 | 1,88 | | 3,02 | | 2,8 | | 3,35 | |

P = poids moyen à J + 8
GP = gain de poids moyen de J - 2 à J + 8

Les résultats montrent qu'à la dose de 40 ppm/jour l'abiérixine augmente la croissance pondérale de coquelets âgés de 14 jours et contaminés par E. tenella quand ce produit est distribué pendant 10 jours.

On a également trouvé, dans le cadre de l'invention, que l'abiérixine et ses sels non toxiques peuvent être utiles comme facteur de croissance en particulier chez les ruminants et à ce titre peuvent être employés en élevage comme additif alimentaire.

Dans ce contexte, on a remarqué que "in vitro" l'abiérixine présente un intérêt tout particulier par le fait qu'elle permet, à la flore ruménale, d'utiliser l'azote des rations alimentaires animales de manière différente de celle de la nigéricine ou de la monensine, deux antibiotiques ionophores connus.

L'addition d'abiérixine au milieu fermentaire diminue notablement la fermentescibilité des sources protéiques étudiées (graine de lupin doux, tourteau d'arachide, tourteau de soya) tout comme la monensine ou la nigéricine.

L'emploi "in vivo" d'abiérixine pourrait donc limiter la dégradation des protéines dans le rumen et ainsi accroître l'apport d'acides aminés d'origine alimentaire dans l'intestin grêle du ruminant.

Par contre, la présence d'abiérixine ne diminue pas significativement la quantité d'azote ammoniacal fixée par les bactéries. Ce composé n'a donc pas l'effet inhibiteur de la monensine ou de la nigéricine sur la synthèse microbienne, effet qui a été décrit avec différents ionophores ou antibiotiques.

En outre, l'efficacité de la synthèse microbienne mesurée en l'absence d'azote protéique, afin de connaître avec exactitude la quantité d'azote fixée par les bactéries n'a diminué que de 10 % en présence d'abiérixine, mais de plus de 30 % avec la monensine ou la nigéricine, ce qui confirme la différence de comportement du composé de l'invention et des composés connus.

L'addition des effets à la fois sur la fourniture d'acides aminés provenant de protéines alimentaires et sur la quantité de protéines microbiennes synthétisées dans le rumen ne pourrait qu'avoir des répercutions positives sur la nutrition azotée du ruminant.

Il s'est avéré également possible d'envisager l'emploi d'abiérixine dans des rations alimentaires contenant de l'azote non protéique utilisable par les bactéries alors qu'une utilisation des antibiotiques ou ionophores connus à ce jour n'est pas très recommandable dens de telle conditions.

L'absence d'effets négatifs de l'abiérixine sur l'activité microbienne ou sur la production de gaz à partir d'azote protéique a également pu être mise en évidence alors que de tels effets négatifs ont été enregistrés en particulier avec la nigéricine.

L'abiérixine, en tant que tacteur de croissance pour ruminants, présente, par conséquent, des avantages indéniables sur la nigéricine ou la monensine puisque le composé de l'invention améliore l'utilisation de l'azote des rations du ruminant en limitant la dégradation des protéines dans le rumen sans réduire l'activité

fermentaire des microbes.

En outre, on mentionnera que l'abiérixine est capable, en association avec certains antibiotiques en particulier les antibiotiques aminoglycosides, de potentialiser l'action de ces composés.

A ce titre, l'abiérixine et ses sels non toxiques, en association avec certains antibiotiques, pourraient être utilisés en médecine humaine.

Comme indiqué précédemment, l'abiérixine et ses sels pharmaceutiquement acceptables sont également capables d'augmenter la cytotoxicité d'immunotoxines c'est-à-dire de conjugués obtenus à partir d'un anticorps, par exemple un anticorps monoclonal, ou un de ses fragments et une toxine, par exemple la chaîne A de la ricine, ou un de ses fragments.

En conséquence, des immunotoxines associées à l'abiérixine ou à un de ses sels pharmaceutiquement acceptables pourront être utilisées pour des applications cliniques notamment pour éliminer les cellules tumorales metastatiques d'échantillons de moëlle osseuse provenant de patients subissant une transplantation autologue et éviter, en conséquence, le renouvellement de la greffe.

Finalement, l'abiérixine et ses sels pharmaceutiquement acceptables ont montré une toxicité faible.

On a trouvé, par exemple, que la toxicité aiguë $DL_{50}$ de l'abiérixine chez la souris par voie orale est comprise entre 600 et 1000 mg/kg alors que, dans les mêmes conditions, la toxicité d'autres polyéthers polycycliques se révèle beaucoup plus importante, de l'ordre de 50 mg/kg ($DL_{50}$ Ionomycine : 50 mg/kg, complexe de monensine* : 43,8 mg/kg, salinomycine* : 50 mg/kg).

A titre indicatif, la salinomycine* et la nigéricine*, par voie intrapéritonéale chez la souris, présentent une $DL_{50}$ respectivement de 18 mg/kg et de 10 à 15 mg/kg.

* : The Merck Index, 10ème édition (1983).

L'abiérixine et ses sels non toxiques peuvent donc être administrés en tant que principe actif médicamenteux sous forme de compositions unitaires convenant à l'administration en thérapie humaine ou vétérinaire ou en tant qu'additif alimentaire à usage vétérinaire.

Sous son aspect le plus général et en tenant compte de l'utilisation finale choisie, les compositions pharmaceutiques ou vétérinaires en question sont préparées en associant le principe actif selon l'invention, avec un véhicule, diluant ou excipient approprié.

A titre d'exemple, une composition thérapeutique anticoccidienne peut être obtenue par addition d'abiérixine ou d'un de ses sels non toxiques, à la ration alimentaire de base en quantité de l'ordre de 0,02 % en poids du mélange final.

Pour ce qui concerne l'unité d'administration celle-ci peut prendre la forme, par exemple, d'un comprimé, d'une dragée, d'une capsule, d'une gélule, d'une poudre, d'une suspension ou d'un sirop pour l'administration orale, d'un suppositoire pour l'administration rectale, d'une solution stérile ou suspension pour l'administration parentérale.

A usage vétérinaire, l'unité d'administration peut en outre, prendre la forme d'un prémélange contenant le principe actif dispersé sur le véhicule ou diluant ou encore la forme d'un supplément de nourriture contenant le principe actif mélangé au véhicule ou diluant.

Dans ces deux derniers cas, un tel véhicule est de préférence un composant alimentaire pour animaux, par exemple avoine, graine de soya, luzerne, froment, résidus de fermentation, coquilles d'huîtres broyées, mélasses, substances végétales comestibles, farine de soya, kaolin, talc, calcaire broyé, etc...

Sous forme de prémélange, des compositions de l'invention destinées à lutter contre la coccidiose peuvent contenir de 10 à 80 % en poids d'abiérixine ou d'un de ses sels non toxiques et de 90 à 20 % de véhicule ou diluant en poids de la composition. Ces prémélanges peuvent être dilués avec un supplément alimentaire pour animaux ou peuvent être ajoutés directement à une ration alimentaire animale de manière à fournir une alimentation thérapeutique qui peut être ingérée par la volaille.

Alternativement, l'abiérixine et ses sels non toxiques peuvent être administrés sous forme d'une solution ou suspension contenant une quantité thérapeutiquement efficace de ce principe actif dans l'eau de boisson de la volaille.

Une telle quantité thérapeutiquement active peut varier de 0,005 à 0,04 % en poids de la quantité de nourriture journalière chez le poulet.

Les exemples, non limitatifs suivants, illustrent l'invention :

**Exemple 1**

Préparation de l'abiérixine et de son sel sodique.

I. Sel sodique de l'abiérixine

1ère préculture

On stérilise un erlenmeyer de 500 ml pendant 20 min. à 118°C. On y place alors 100 ml de milieu I (30 g de farine de soya, 20 g de glucose, 2,5 g de corn-steep, 1,0 g de $K_2HPO_4$, 1000 ml d'eau, le pH étant ajusté à 7,2 avec NaOH 10N). On introduit alors un tube de spores congelés de Streptomyces C.N.C.M.-I-321 et on agite l'ensemble dans une secoueuse animée d'un mouvement rotatif pendant 3 jours à 27°C (vitesse : 200 tr/min.).

2ème préculture

Sous conditions stériles, on transfère la 1ère préculture dans un fermenteur de 20 l contenant 1 l de milieu I

puis on agite pendant 24h.

A. Production

On stérilise un fermenteur de 20 l contenant 15 l de milieu II (extrait de levure : 1 g, extrait de viande : 1 g, "casaminoacids" (caséine hydrolysée par un acide) 4 g, glucose : 15 g, oléate de méthyle : 20 g, eau : 1000 ml, le pH étant ajusté à 7,2 avec NaOH 10N) pendant 45 min. à 188°C. On y transfère alors la 2ème préculture et on maintient le milieu ainsi formé en culture durant 7 jours à la température de 27°C sous agitation (vitesse 650 tr/min) et sous aération de 0,75 l d'air /min/l de milieu.

B. Purification

a) Centrifugation et extraction

En fin de culture en fermenteur, on sépare le mycélium du jus de fermentation par centrifugation à 40 000 g. On place, dans 500 ml d'éthanol, le mycélium récolté dans le cylindre de la centrifugeuse et on le broie très finement afin de permettre une meilleure action du solvant d'extraction. Après broyage, on ajoute 4 l d'éthanol (300 ml d'éthanol/l de milieu centrifugé) et on laisse agir le solvant pendant 2 h. On sépare alors le mycélium sur filtre en verre fritté et on concentre l'extrait éthanolique à l'aide d'un évaporateur cyclone. On poursuit ensuite la concentration au moyen d'un évaporateur rotatif jusqu'à obtention d'une huile rouge foncé. On ajoute ensuite 500 ml d'acide chlorhydrique 0,1N, ce qui permet une meilleure extraction à l'éther éthylique (3 à 4 fois, 250 ml d'éther). On sèche sur sulfate de sodium anhydre et on concentre à l'évaporateur rotatif. On analyse alors l'extrait éthéré par chromatographie sur couche mince de gel de silice, ce qui permet de différencier l'abiérixine d'autres polyéthers polycycliques formés au cours de la fermentation (nigéricine).

Valeur des Rfs

| | Couleur révélée à la vanilline | Eluant* |
|---|---|---|
| Acide gras | Bleu | 0,70 |
| Nigéricine | Rouge foncé | 0,47 |
| Abiérixine | Marron rougeâtre | 0,29 |

* : chloroforme/méthanol 90/10

b) Purification

On réalise 3 chromatographies successives de l'extrait éthéré à savoir :

1) sur colonne de gel de silice (diamètre des particules : 60 à 200 µm)

L'on utilise à cet effet deux éluants :

- cyclohexane/acétate d'éthyle 7/3 puis on fait varier régulièrement le pourcentage d'acétate d'éthyle jusqu'à 100 %.
- chloroforme/méthanol 9/1 puis on fait varier le pourcentage de méthanol jusqu'à 100 %.

Cette première colonne permet d'éliminer les acides gras et une grande partie de la nigéricine.

2) Chromatographie éclair

On traite alors les fractions enrichies en abiérixine par chromatographie éclair qui utilise des caractères propres à la chromatographie sous pression. Il s'agit d'une technique rapide et économique permettant en 10 à 15 min. la séparation d'échantillons dont les poids sont compris entre 0,04 et 2,5 g.

On choisit à cet effet un solvant qui fasse migrer l'élément désiré à un Rf de 0,35 en chromatographie sur couche mince. On remplit une colonne de diamètre approprié jusqu'à une hauteur de 15 cm avec du gel de silice (0,040-0,063 mm),on ajoute l'éluant et on règle la pression pour avoir un debit de $\pi^3$ $cm^3$/min. ($\pi$ : rayon de la colonne en cm).

En fonction du diamètre de la colonne et du volume d'éluant on a donné dans le tableau ci-dessous les quantités d'abiérixine et de nigéricine dans les fractions collectées ainsi que les Rf respectifs.

| Diamètre de la colonne (mm) | Volume d'éluant (ml) | Echantillon charge typique (mg) | | Volume des fractions collectées (ml) |
|---|---|---|---|---|
| | | Rf : 0,2 | Rf : 0,1 | |
| 10 | 160 | 100 | 40 | 5 |
| 20 | 200 | 400 | 160 | 10 |
| 30 | 400 | 900 | 360 | 20 |
| 40 | 600 | 1600 | 600 | 30 |
| 50 | 1000 | 2500 | 1000 | 50 |

On utilise 3 éluants successifs à savoir :

| | |
|---|---|
| - chloroforme pur | 500 ml |
| - chloroforme/méthanol 9,8/0,2 | 300 ml |
| - chloroforme/méthanol 9,6/0,4 | 500 ml |

Les volumes d'éluants donnés ci-dessus permettent d'éliminer la totalité de l'élaiophyline et une partie de la nigéricine formées au cours de la fermentation.

3) Chromatographie liquide haute pression préparative

On effectue la dernière chromatographie en CLHP en utilisant deux colonnes de gel de silice branchées en série. On emploie à cet effet des colonnes de 40 cm de longueur et 6 cm de diamètre et on effectue la détection à l'aide d'un réfractomètre différentiel intégré au chromatographe.

On utilise deux éluants et deux valeurs de débit :

1er éluant : 3l de chloroforme/méthanol 95/5 (v/v). Le débit est de 50 ml/min. jusqu'à l'élution des pics antérieurs à l'abiérixine.

2ème éluant : 4 à 5l de chloroforme/méthanol 90/10. On règle le débit à 100 ml/min. et on recycle une fois la fraction contenant le composé désiré.

On collecte des fractions de 100 ml puis on évapore le solvant.

De cette manière, on obtient le sel sodique de l'abiérixine sous forme d'une mousse blanche solide.

P.F. : 120-123° C.

II. Abiérixine

On peut obtenir l'abiérixine monohydratée à partir de son sel de sodium en modifiant les éluants au niveau de la chromatographie éclair (paragraphe 2) en leur ajoutant 0,5 % d'acide acétique, à savoir :

| | |
|---|---|
| - chloroforme/acide acétique 99,5/0,5 | 500 ml |
| - chloroforme/méthanol/acide acétique 95,75/1,75/0,5 | 500 ml |
| - chloroforme/méthanol/acide acétique 95,75/3,75/0,5 | 500 ml |

De cette manière, on obtient l'abiérixine monohydratée.

P.F. : 83-85° C

**Exemple 2**

On a préparé un prémélange alimentaire pour animaux en mélangeant intimement les ingrédients suivants :

| | Ingrédients | Parties en poids |
|---|---|---|
| 1) | Abiérixine | 50 |
| | Granulés de luzerne | 50 |
| 2) | Abiérixine | 40 |
| | Mélasses solubles | 60 |
| 3) | Abiérixine | 30 |
| | Grains séchés de mais | 70 |
| 4) | Sel de sodium d'abiérixine | 40 |
| | Parine de maïs | 30 |
| | Grains séchés de maïs | 30 |
| 5) | Sel de sodium d'abiérixine | 60 |
| | Graines moulues de soya | 40 |
| 6) | Sel de sodium d'abiérixine | 20 |
| | Grains séchés de mais | 60 |
| | Son de froment | 20 |
| 7) | Abiérixine | 80 |
| | Son de froment | 20 |
| 8) | Abiérixine | 25 |
| | Graines de soya | 75 |

**Revendications**

1. Ionophore polyéther répondant à la formule :

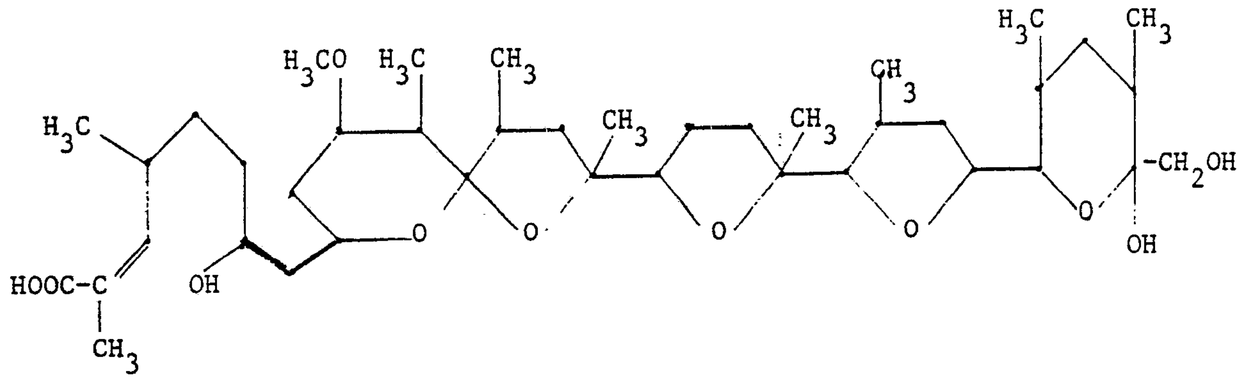

sous forme anhydre ou hydratée ainsi que les sels non toxiques dudit ionophore polyéther.

2. Ionophore polyéther selon la Revendication 1, sous la forme de son monohydrate présentant les caractéristiques suivantes :

- un point de fusion de 83-85° C.
- une masse moléculaire de 742
- un $\alpha_J^{25}$ de +45° (c = 0,03 n, méthanol)
- une solubilité dans les alcools inférieurs, les esters inférieurs, l'éther éthylique, le chloroforme et à 40 % dans l'eau;
- un spectre infra-rouge dans le KBr avec les bandes caractéristiques suivantes : 3360, 2980, 2940, 2880, 2640, 1730, 1640, 1460, 1370, 1360, 1355, 1310, 1300, 1290, 1260, 1240, 1205, 1185, 1115, 1080, 1070, 1040, 1030, 1020, 990, 980, 960, 930, 910, 880, 870, 845, 810, 730, 715, 690, 640, 620, 610, 590, 535, 500, 460, 455, 415, 395 $cm^{-1}$
- un spectre ultra-violet dans le cyclohexane avec une bande intense ($\lambda$ max = 217 nm, $\varepsilon$ : 10400) et une bande faible ($\lambda$ max = 275 nm, $\varepsilon$ = 2800).

3. Ionophore polyéther selon la Revendication 1, sous la forme de son sel de sodium présentant un point de fusion de 120-123° C.

4. Procédé de préparation de l'ionophore polyéther selon la Revendication 1, caractérisé en ce que l'on cultive Streptomyces C.N.C.M.-I-321 ou Streptomyces hygroscopicus NRRL 3602 en milieu nutritif contenant une source assimilable de carbone, d'azote et de sels minéraux puis l'on fait éventuellement réagir le composé obtenu sous forme acide avec une base organique ou inorganique appropriée pour former un sel non toxique de ce composé. Composition pharmaceutique ou vétérinaire caractérisée en ce qu elle contient comme principe actif essentiel au moins un des composés définis à la Revendication 1, en association avec un véhicule, diluant ou excipient approprié.

6. Composition vétérinaire ayant une activité anticoccidienne, caractérisée en ce qu'elle contient comme principe actif essentiel au moins un des composés définis à la Revendication 1, en association avec un véhicule, diluant ou excipient approprié.

7. Composition vétérinaire alimentaire utile comme facteur de croissance, caractérisée en ce qu'elle contient comme principe actif essentiel au moins un des composés définis à la Revendication 1, en association avec un véhicule, diluant ou excipient approprié.

8. Composition pharmaceutique capable d'exalter l'activité cytotoxique d'immunotoxines, caractérisée en ce qu'elle contient comme principe actif essentiel au moins un des composés définis à la Revendication 1, en association avec un véhicule, diluant ou excipient approprié.

9. Microorganisme de souche Streptomyces déposé sous le numéro C.N.C.M. -I-321.

**Patentansprüche**

1. Ionophorer Polyether entsprechend der Formel :

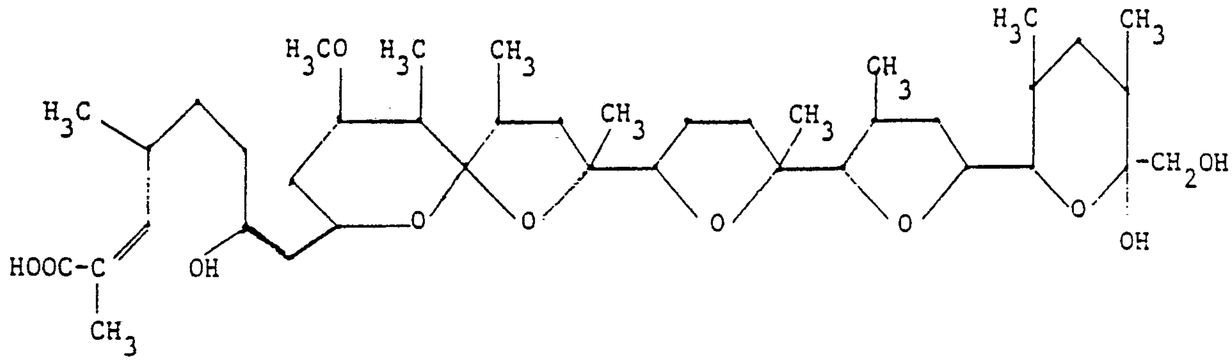

in wasserfreier oder hydratisierter Form sowie die nicht-toxischen Salze dieses ionophoren Polyethers.

2. Ionophorer Polyether nach Anspruch 1 in Form seines Monohydrates mit den folgenden Eigenschaften : F. 83-85°C; Molekulargewicht 742; $\alpha_J^{25} = +45°$ (c = 0,03 n, Methanol); Löslichkeit in niederen Alkoholen, niederen Estern, Ethylether, Chloroform und zu 40 % in Wasser; IR-Spektrum in KBr mit den folgenden charakteristischen Banden : 3360, 2980, 2940, 2880, 2640, 1730, 1640, 1460, 1370, 1360, 1355, 1310, 1300, 1290, 1260, 1240, 1205, 1185, 1115, 1080, 1070, 1040, 1030, 1020, 990, 980, 960, 930, 910, 880, 870, 845, 810, 730, 715, 690, 640, 620, 610, 590, 535, 500, 460, 455, 415, 395 $cm^{-1}$; UV-Spektrum in Cyclohexan mit einer starken Bande ($\lambda$ max = 217 nm, $\varepsilon$ : 10400) und einer schwachen Bande ($\lambda$ max = 275 nm, $\varepsilon$ = 2800).

3. Ionophorer Polyether nach Anspruch 1 in Form seines Natriumsalzes mit einem Schmelzpunkt von 120-123°C.

4. Verfahren zur Herstellung eines ionophoren Polyethers nach Anspruch 1, dadurch gekennzeichnet, daß man Streptomyces C.N.C.M.-I-321 oder Streptomyces hygroscopicus NRRL 3602 in einem Nährmedium, das eine Quelle von assimilierbarem Kohlenstoff, Stickstoff und Mineralsalzen enthält, züchtet, worauf man gegebenenfalls die in saurer Form erhaltene Verbindung mit einer entsprechenden organischen oder anorganischen Base umsetzt, um ein nicht-toxisches Salz dieser Verbindung zu bilden.

5. Pharmezeutische oder veterinärmedizinische Zusammensetzung, dadurch gekennzeichnet, daß sie als wesentlichen Wirkstoff mindestens eine der in Anspruch 1 definierten Verbindungen zusammen mit einem geeigneten Medium, Verdünnungsmittel oder Träger enthält.

6. Veterinärmedizinische Zusammensetzung mit Anticoccidien-Aktivität, dadurch gekennzeichnet, daß sie als wesentlichen Wirkstoff mindestens eine der in Anspruch 1 definierten Verbindungen zusammen mit einem geeigneten Medium, Verdünnungsmittel oder Träger enthält.

7. Als Wachstumsfaktor geeignete, veterinärmedizinische, alimentäre Zusammensetzung, dadurch gekennzeichnet, daß sie als wesentlichen Wirkstoff mindestens eine der in Anspruch 1 definierten Verbindungen zusammen mit einem geeigneten Medium, Verdünnungsmittel oder Träger enthält.

8. Pharmazeutische Zusammensetzung, die die cytotoxische Aktivität von Immunotoxinen steigern kann, dadurch gekennzeichnet, daß sie als wesentlichen Wirkstoff mindestens eine der in Anspruch 1 definierten Verbindungen zusammen mit einem geeigneten Medium, Verdünnungsmittel oder Träger enthält.

9. Mikroorganismus vom Stamm Streptomyces, hinterlegt unter der Nummer C.N.C.M.-I-321.

## Claims

1. Polyether ionophore corresponding to the formula :

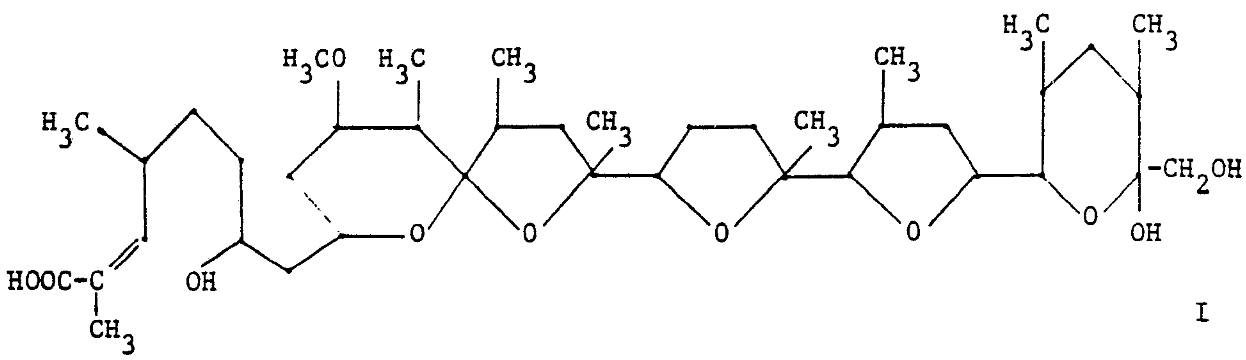

I

in anhydrous or hydrated form and pharmaceutically acceptable salts thereof.

2. Polyether ionophore according to Claim 1 in monohydrated form presenting the following characteristics :
- a melting point of 83-85° C
- a molecular mass of 742
- a $\alpha_J^{25}$ of +45° (C=0.03 n, methanol)
- solubility in lower alcohols, lower esters, ethyl ether, chloroform and of 40 % in water ;
- an infra-red spectrum in KBr with the following characteristic bands : 3360, 2980, 2940, 2880, 2640, 1730, 1640, 1460, 1370, 1360, 1355, 1310, 1300, 1290, 1260, 1240, 1205, 1185, 1115, 1080, 1070, 1040, 1030, 1020, 990, 980, 960, 930, 910, 880, 870, 845, 810, 730, 715, 690, 640, 620, 610, 590, 535, 500, 460, 455, 415, 395 $cm^{-1}$
- an ultra-violet spectrum in cyclohexane with a large band (λ max = 217 nm, ε =10400) and a weak band (λ max=27 nm, ε = 2800).

3. Polyether ionophore according to Claim 1 in the form of its sodium salt presenting a melting point of 120-123° C.

4. Process for preparing the polyether ionophore according to Claim 1, whereby Streptomyces C.N.C.M.-I-321 or Streptomyces hygroscopus NRRL 3602 is cultivated in a nutrient medium and the compound so obtained in acid form is optionally reacted with an appropriate organic or inorganic base to form a phanmaceutically acceptable salt of this compound.

5. Pharmaceutical or veterinary composition containing as essential active ingredient at least one compound according to Claim 1, in association with an appropriate carrier, diluent or excipient.

6. Veterinary composition having an anticoccidia action containing as essential active ingredient at least one compound according to Claim 1, in association with an appropriate carrier, diluent or excipient.

7. Veterinary feed composition useful as growth promoter containing as essential active ingredient at least one compound according to Claim 1, in association with an appropriate carrier, diluent or excipient.

8. Pharmaceutical composition capable of increasing the cytotoxicity of immunotoxins containing as essential active ingredient at least one compound according to Claim 1, in association with an appropriate carrier, diluent or excipient.

9. Microorganism of the Streptomyces genius deposited under No. C.N.C.M.-I-321.

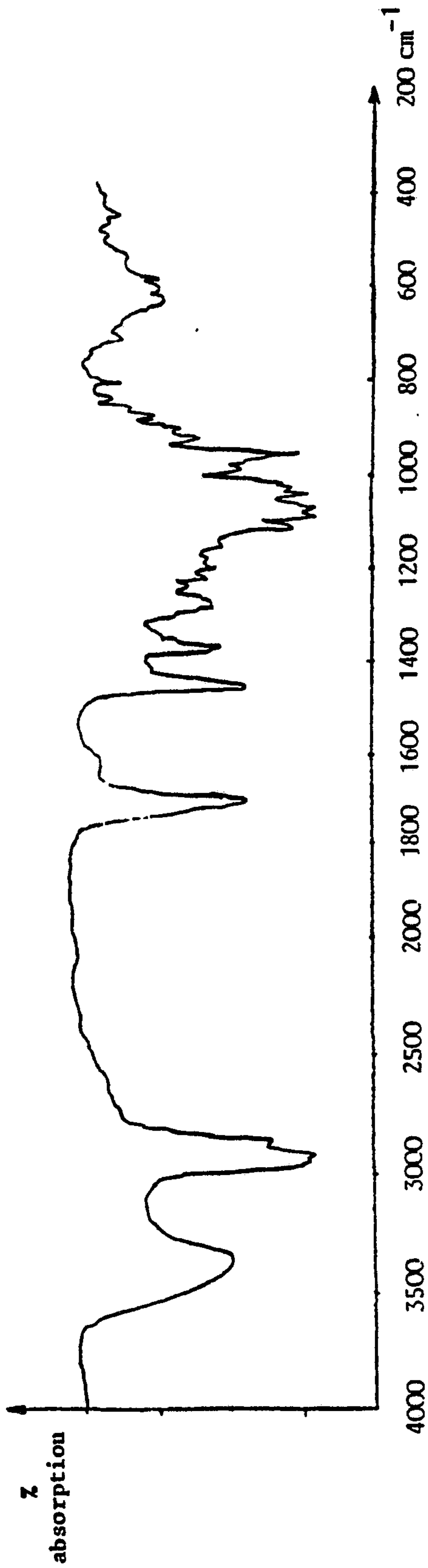


Figure 1

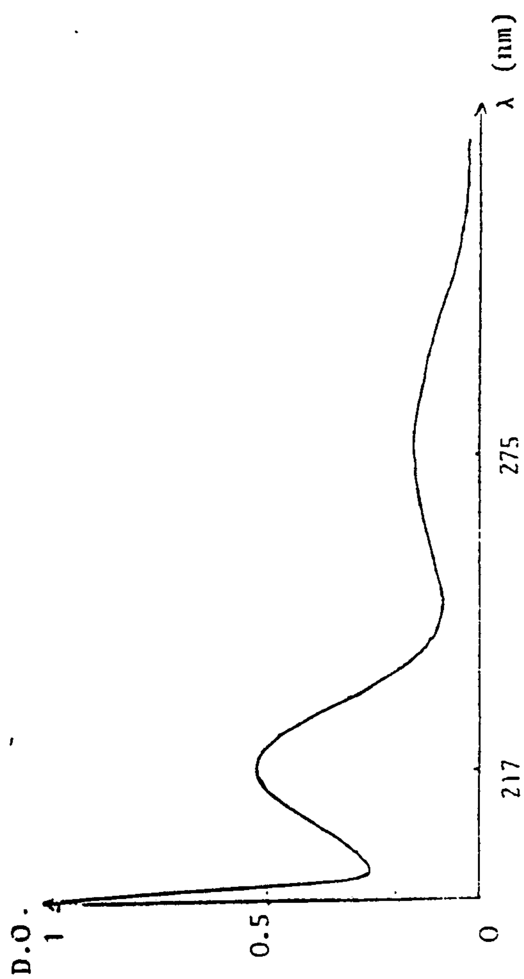


Figure 2

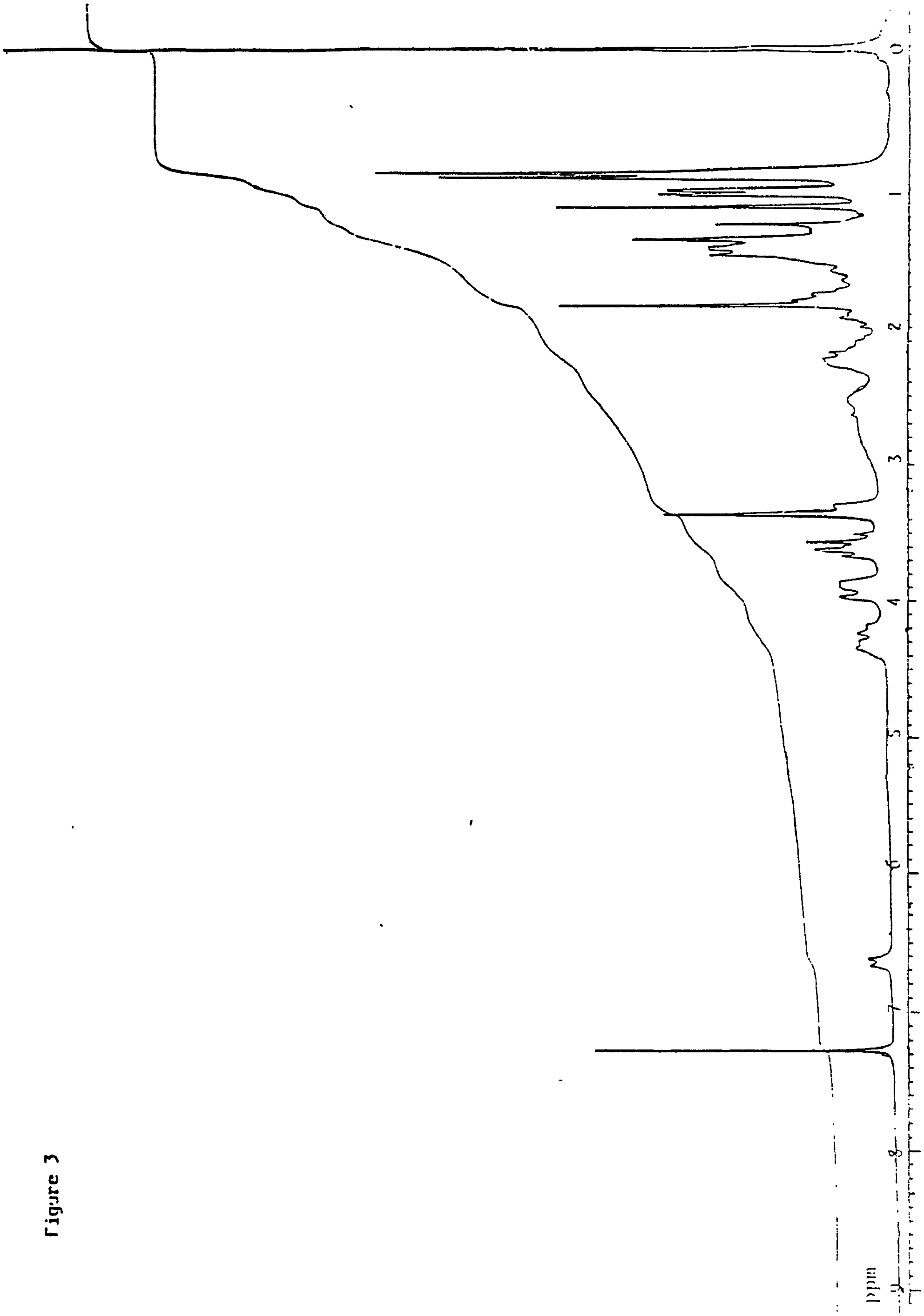


Figure 3

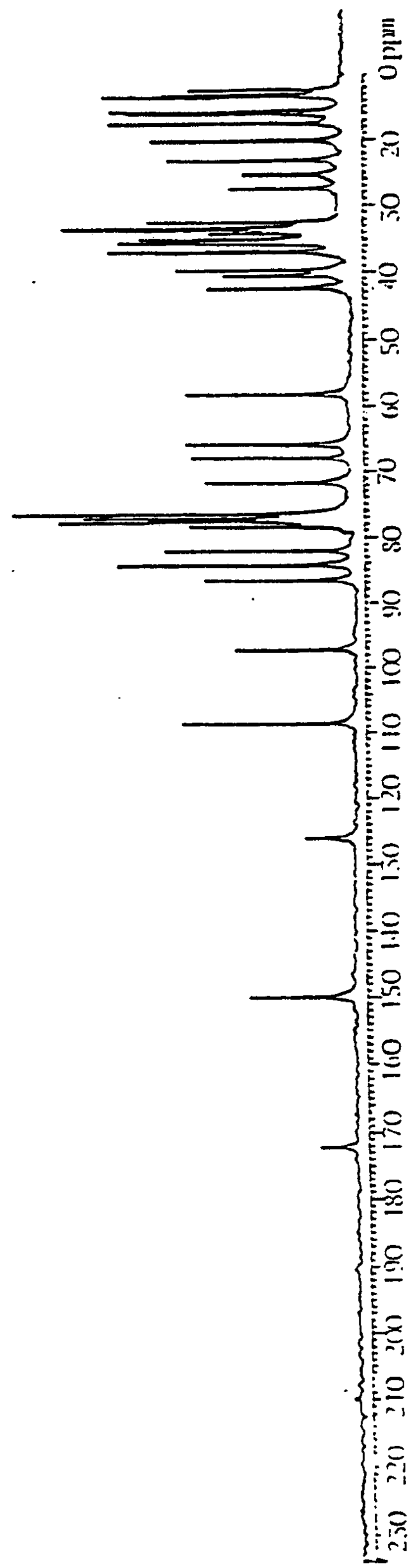

Figure 4

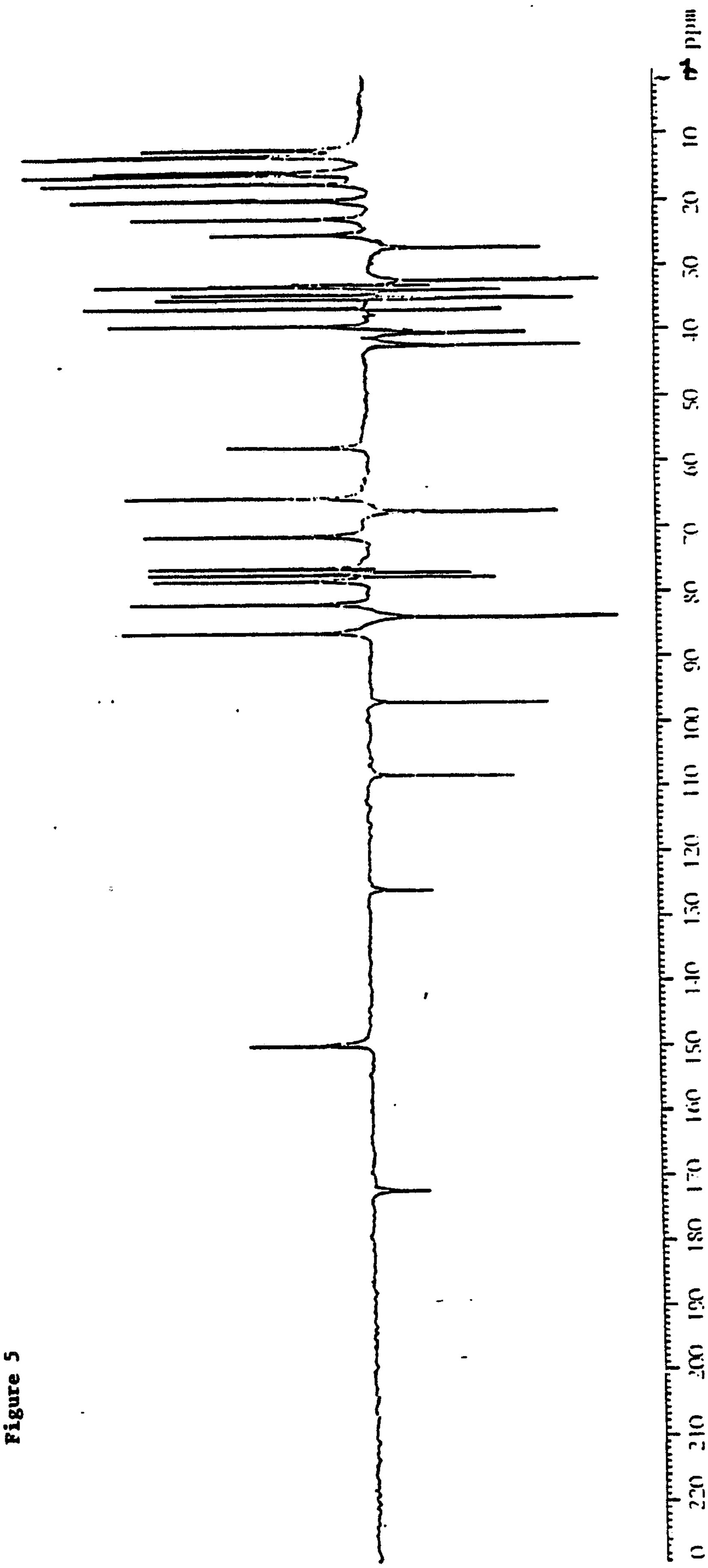
ppm
0
10
20
30
40
50
60
70
80
90
100
110
120
130
140
150
160
170
180
190
200
210
220
230

Figure 5